Europäisches Patentamt

(19) **European Patent Office**

Office européen des brevets

(11) Publication number: **0 158 372**
**A1**

(12) # EUROPEAN PATENT APPLICATION

(21) Application number: **85200065.2**

(22) Date of filing: **23.01.85**

(51) Int. Cl.⁴: **A 61 N 1/14**

(30) Priority: **07.02.84 NL 8400373**

(43) Date of publication of application:
**16.10.85 Bulletin 85/42**

(84) Designated Contracting States:
**AT BE CH DE FR GB IT LI LU NL SE**

(71) Applicant: **Hepag Health Education Products Ltd.**
**Seebahnstrasse 31**
**Zürich(CH)**

(72) Inventor: **Van Baarle, Johannes Gerardus Antonius**
**Willemsparkweg 194**
**NL-1071 HW Amsterdam(NL)**

(74) Representative: **de Boer, Hindrik Geert Jan et al,**
**De Boer & Wigman De Lairessestraat 133**
**NL-1075 HJ Amsterdam(NL)**

(54) **Covering piece and material for therapeutic purposes.**

(57) The invention relates to a cover or shield for therapeutic purposes consisting of one or more layers of insulating material in which at least two electrodes are embedded, which electrodes are interconnected through electrical conductors. If the cover or shield is positioned in close proximity parallel to a human or animal body it will form an equipotential surface parallel to that body.

EP 0 158 372 A1

COVERING PIECE AND MATERIAL

It is well known, that through different causes the human and animal body can show signs of lack of health. This can go with unfavourable tension of muscle groups and/or disturbed circulation of blood in skin and underlying tissues.

It is also known, that these symptoms can be relieved by balancing body surface tension between certain spots on the skin.

Thus, European patent application 0107258 describes a garment or belt, provided with metallic electrodes, designed to be in direct physical contact with the skin, which electrodes are arranged so as to touch the acupuncture spots on the skin, and which electrodes are interconnected by means of conductive wires or strips.

However, the use of uncovered electrodes poses some applicational restrictions. Thus, some people are allergic to direct contact with metal surfaces, such as copper, brass, aluminium and nickel.

It has now been found, that the symptoms of ill health can also be relieved by means of a therapeutic shield or cover, consisting of one or more layers of insulating material, in which two or more electrodes are embedded, the electrodes being interconnected by means of an electric conductor, the shield or cover being designed in such a manner, that it may be positioned in close proximity substantially parallel to the body surface. It has appeared, that by means of this construction it is possible to establish an equipotential surface in the shield or cover without the use of any external source of electricity, which equipotential surface is substantially parallel to the body surface, whereby the symptoms of ill health are relieved.

The terms "shield" or "cover" are used to denote generally any object intended to be for therapeutic purposes for some length of time in close proximity to the human or animal body, such as a single or multilayered piece of clothing, a facial mask, an inlay sole, a piece of furniture or a preformed or elastic cover for a piece of furniture. The material can be woven or knitted or made from so-called stretch materials. It can also be made from plastic, rubber, leather, or similar materials.

The term "therapeutic purposes", apart from referring to the recovery of human or animal ailments, includes also the alleviation of bodily discomfort and the improvement of the feeling of well-being, e.g. as a result of the disappearance of a feeling of tiredness, or nervous strain.

The shield or cover according to the invention contains at least two electrodes, embedded in the sheet of electrically insulating material. The electrodes may be manufactured from sheet or gauze material. The gauze material can be braided or woven from wire, strip metal or multiple twisted wire. The sheet material can be provided with mechanical strengthening means on the side away from the body, with protrusions to fix the conductor to the layer of insulating material.

The diameter of the electrode is usually about 1 cm, but it may be larger, e.g. 3-12 cm, depending on the intended use of the shield or cover. The electrodes may have the shape of e.g. a cross, a ring, a spiral or a wire wound as a spiral. The thickness of the material, from which the electrode is manufactured, depends on the strength and the flexibility required for the particular purpose. For sheet material this is for instance between 0,05 and 1,5 mm. In the case of a gauze material the space between the wires is not necessarily uniform.

The electrodes may be connected by several parallel conductors. The material can be copper, brass or another well conductive material. If desired, electrodes and interconnecting conductors can be formed from one single sheet, for instance by means of punching.

The position of the electrodes can be adapted to the size and the shape of the body, for which it is designed. The exact positioning of the electrodes can be based on research on the individual for which it is being designed, or on epidemological research.

For more general applications the shield or cover according to the invention is of substantially fixed shape and dimensions. Special reinforcing material may be used to realize this effect, such as leather and hard PVC. It has been found that these articles are easy to manufacture, e.g. in mass production. Moreover, the electrical connection is independent of the frequency of use of the shield or cover.

For most applications, the position of the electrodes is irregular and non-periodic. However, the electrodes can also be placed in a mattress deck in a matrix of e.g. 15 by 15 cm, in rows, of 4 across and 5 in the length of the body.

An example of a suitable embodiment of the invention is a bucket chair seat. In this case there are for instance five electrodes that have been put between two cloth layers. For instance there are two electrodes where the lower edge of the buttocks would be seated, each of them symmetrical to the middle of the buttock concerned. The third electrode will be positioned opposite the coccyx and be connected with the first and second. The fourth electrode will be opposite the connection of sacrum and fifth lumbar vertebra, and is connected to the third electrode. The fifth electrode is situated between the first lumbar and the twelfth thoracic vertebra and is connected to the fourth electrode. The electrodes may have a diameter of 3-12 cm, preferably 5-10 cm. The chair seat can be executed as a loose cloth element, as a cover, or as an inlay. The chair seat can also be rigidly integrated with the electrodes in place, for instance in a bucket chair. The chair can also be designed as a bench or a carseat.

It is known, that certain ailments find expression in certain characteristic features in e.g. the face, the palm of a hand, or the sole of a foot. For these reasons it is possible to analyse a photographic portrait, or a print of a hand or a foot to discover such characteristic features and then to design a cover or a shield according to the invention to remedy the shortcomings found in the analysis. For use in connection with a foot one will use an inlay sole provided with electrodes according to the invention; for a face, a facial mask similarly designed, will provide the desired relief from the symptoms of the ailment. The electrodes used in an inlay sole or a facial mask usually have a diameter of approximately 1 cm.

CLAIMS

1.  Cover or shield for therapeutic purposes, consisting of one or more layers of insulating material, characterized in that embedded in the layer of insulating material at least two electrodes of electrically conductive material are provided, which electrodes are interconnected through an electrical conductor, the shield or cover being designed in such a manner, that if it is positioned in close proximity to a human or animal body, it will form an equipotential surface substantially parallel to this body.

2.  Cover or shield according to claim 1, characterized in that the electrodes are manufactured from sheet metal.

3.  Cover or shield according to claim 1, characterized in that the electrodes are manufactured from gauze material.

4.  Cover or shield according to claims 1-3, characterized in that it is substantially of fixed shape and dimensions.

5.  Cover or shield according to claims 1-4, characterized in that it is a bucket chair seat, or a cover for such a seat, provided with electrodes having a diameter of 3-12, preferably of 5-10 cm.

6.  Cover or shield according to claims 1-4, characterized in that it is an inlay sole, provided with electrodes having a diameter of about 1 cm.

0158372

7. Cover or shield according to claims 1-4, <u>charac-
terized in</u> that it is a facial mask, provided with
electrodes having a diameter of about 1 cm.

8. Material for the manufacture of a cover or shield
for therapeutic purposes according to any one or
more of the preceding claims, consisting of one or
more layers of insulating material, <u>characterized in</u>
that embedded in the layer of insulating material at
least two electrodes of electrically conductive
material are provided, which electrodes are interconnected through an electrical conductor, in order
to form an equipotential surface substantially
parallel to the body in connection with which it is
going to be used.

## European Patent Office

## EUROPEAN SEARCH REPORT

EP 85 20 0065

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | FR-A- 429 322 (RINGELMANN) <br> * Page 1, lines 30-40; figure 1 * | 1,2 | A 61 N 1/14 |
| A | | 4,8 | |
| Y | FR-A-1 541 165 (YALLOUZ) <br> * Page 1, paragraphs 4,9 and right-hand column, paragraph 10 * | 1 | |
| A | GB-A-2 025 237 (OLIVER) <br> * Page 1, line 16 - page 2, line 14 * | 1-6 | |
| A | DE-C- 813 194 (HELMLE) <br> * Page 1, lines 19-28 * | 2,3 | |
| A | GB-A- 334 774 (BALLANTYNE) <br> * Page 1, lines 14-26 * | 2,3 | TECHNICAL FIELDS SEARCHED (Int. Cl.4) <br><br> A 61 N |
| A | CH-A- 196 814 (GUGGENHEIM) <br> * Page 2, paragraph 2 and the last two paragraphs * | 5,6 | |
| A | US-A-3 971 387 (MANTELL) <br> * Column 2, lines 22-30 * | 4,7 | |
| P,X | EP-A-0 107 258 (HEPAG) <br> * Page 3, lines 18-28; claim 1 * | 1,8 | |

The present search report has been drawn up for all claims

| Place of search | Date of completion of the search | Examiner |
|---|---|---|
| THE HAGUE | 18-06-1985 | SIMON J.J.E. |